Europäisches Patentamt

European Patent Office

Office européen des brevets

(19)

(11) EP 0 804 512 B1

(12) EUROPÄISCHE PATENTSCHRIFT

(45) Veröffentlichungstag und Bekanntmachung des Hinweises auf die Patenterteilung:
07.01.1999 Patentblatt 1999/01

(21) Anmeldenummer: 96900538.8

(22) Anmeldetag: 16.01.1996

(51) Int Cl.⁶: **C09C 1/64**, C09D 7/12, C09D 5/10, C08K 9/04, C08K 9/02, C09D 11/00, A61K 7/00

(86) Internationale Anmeldenummer:
PCT/DE96/00085

(87) Internationale Veröffentlichungsnummer:
WO 96/22336 (25.07.1996 Gazette 1996/34)

(54) **FARBIGE ALUMINIUMPIGMENTE, VERFAHREN ZU DEREN HERSTELLUNG SOWIE DEREN VERWENDUNG**

COLOURED ALUMINIUM PIGMENTS, PROCESS FOR PRODUCING THEM AND THEIR USE

PIGMENTS D'ALUMINIUM COLORES, LEUR PROCEDE DE PRODUCTION ET LEUR UTILISATION

(84) Benannte Vertragsstaaten:
DE ES FR GB IT

(30) Priorität: 18.01.1995 DE 19501307

(43) Veröffentlichungstag der Anmeldung:
05.11.1997 Patentblatt 1997/45

(73) Patentinhaber: ECKART-WERKE STANDARD BRONZEPULVER-WERKE CARL ECKART GMBH & CO.
D-90763 Fürth (DE)

(72) Erfinder:
• REISSER, Wolfgang
D-91220 Schnaittach (DE)
• MEBAREK, Denise
D-91257 Büchenbach (DE)

(74) Vertreter:
LOUIS, PÖHLAU, LOHRENTZ & SEGETH
Postfach 3055
90014 Nürnberg (DE)

(56) Entgegenhaltungen:
EP-A- 0 266 247    EP-A- 0 328 906
EP-A- 0 571 836    EP-A- 0 580 022
US-A- 2 071 156    US-A- 3 026 220
US-A- 3 067 052    US-A- 4 158 074
US-A- 5 261 955

• DATABASE WPI Week 8930 Derwent Publications Ltd., London, GB; AN 89-216528 & JP,A,01 153 761 (MITSUBISHI MOTOR), 15.Juni 1989

• DATABASE WPI Week 8513 Derwent Publications Ltd., London, GB; AN 85-077466 & JP,A,60 029 402 (PENTEL), 14.Februar 1985

**Beschreibung**

Die Erfindung betrifft farbige Aluminiumpigmente, ein Verfahren zu ihrer Herstellung sowie deren Verwendung.

Aluminiumpigmente werden in großem Umfang in Beschichtungen als Effektpigmente eingesetzt. Unter Effektpigmenten versteht man Pigmente, die eine gerichtete Reflexion an überwiegend flächig ausgebildeten, orientierten metallischen oder stark lichtbrechenden Partikeln aufweisen (DIN 55944). Sie sind stets plättchenförmig und haben verglichen mit Farbpigmenten sehr große Partikeldurchmesser. Ihre optischen Eigenschaften werden durch Reflexion und Interferenz bestimmt. Je nach Transparenz, Absorption, Dicke, Ein- oder Mehrschichtenaufbau zeigen die Effektpigmente metallischen Glanz, Perlglanz, Interferenz oder Interferenzreflexion. Hauptanwendungsbereich sind Kosmetik und Automobilsektor, daneben Kunststoffeinfärbung, Anstrichmittel, Lederbeschichtungen, Druckindustrie und Keramikindustrie. (Für eine umfassende Darstellung des technischen Hintergrunds vgl. W. Ostertag, Nachr. Chem. Tech. Lab. 1994, 9, 849.)

Die am häufigsten verwendeten Effektpigmente sind Aluminiumflakes und beschichtete Glimmerplättchen, wobei Aluminiumpigmente typischen Metallglanz, beschichtete Glimmerplättchen typischen Perlglanz zeigen.

In den vergangenen Jahren hat der Bedarf an farbigen Effektpigmenten stark zugenommen. Daher wurden beispielsweise oxidbelegte Kupfer- und Messingplättchen, mit Übergangsmetalloxiden beschichtete Substrate wie Muskovit, Phlogopit oder Glas, Guanin-Einkristalle (Fischsilber), BiOCl-Einkristalle, plättchenförmige Hämatit-Einkristalle, plättchenförmige Phtalocyanine oder zerkleinerte dünne Mehrschichtenfilme mit Fabry-Perot-Struktur als Effektpigmente eingesetzt.

Um Farbeffekte zu erzielen, werden u.a. auch Aluminiumpigmente mit transparenten Farbpigmenten gemischt. Die koloristischen Möglichkeiten sind bei dieser Methode allerdings insofern begrenzt, als auf diese Weise keine Interferenzeffekte erzielt werden können, die Pigmente also keinen Perlglanz aufweisen. Interferenzpigmente mit Perlglanz, die meist auf beschichteten Glimmerplättchen basieren, weisen jedoch aufgrund ihrer Transparenz eine schlechtere Deckfähigkeit auf als Aluminiumpigmente. Man hat daher versucht, durch Einfärben von Aluminiumpigmenten Pigmente mit der guten Deckfähigkeit der Aluminiumflakes und den koloristischen Möglichkeiten der Interferenzpigmente herzustellen.

US-4,328,042 und EP-A-0 033 457 beschreiben die Herstellung goldfarbener Aluminiumpigmente durch Abscheidung von Eisenoxid, wobei Eisenpentacarbonyl in einer durch Fluidisierung mit Inertgas erzeugten Wirbelschicht der Aluminiumflakes mit Sauerstoff oxidiert wird. Der Nachteil dieses Verfahrens ist der sehr große technologische Aufwand.

US-5,037,475 beschreibt die Herstellung von Aluminiumpigmenten, die durch Fixierung von Farbpigmenten auf der Metalloberfläche eingefärbt sind. Die Fixierung der Farbpigmente erfolgt über carboxylgruppenhaltige Polymere. Zur Haftungsverbesserung kann durch Polymerisation eine Schutzschicht aufgebracht werden. Die so erzeugten Pigmente weisen jedoch nur eine geringe Farbintensität auf.

WO 91/04293 (PCT/US90/05236) beschreibt die Einfärbung von Aluminiumpigmenten durch Fixierung von polymerbeschichteten Farbpigmenten aus wässerigen Lösungsmitteln über elektrostatische Kräfte auf den Metalloberflächen. Das Beschichtungsergebnis hängt dabei in komplexer Weise von der Aluminiumpigmenttype, der Art der Polymerbeschichtung der Farbpigmente, der Lösungsmittelzusammensetzung und dem pH-Wert ab.

EP-A-0 328 906 offenbart Titandioxid-beschichtete Metallpigmente, u.a. auch Aluminiumpigmente, deren Herstellung durch Hydrolyse einer organischen Titanatester-Verbindung, z.B. Tetraisopropoxytitan, in Gegenwart der in einem organischen Medium suspendierten Metallplättchen bei pH 4 bis 8 erfolgt. Verschiedene Farbtöne lassen sich mit diesem Verfahren durch Variation der Dicke der Titandioxidschicht erzielen. Für die Herstellung der beschichteten Pigmente ist die Einhaltung spezifischer Bedingungen entscheidend. Der pH muß im Bereich von 4 bis 8 liegen, und die Zutropfgeschwindigkeit bei der Zugabe des Titanatesters muß im Bereich von 1,0 x $10^{-7}$ bis 1,0 x $10^{-4}$ Mol pro Minute und $m^2$ Metalloberfläche liegen. Daher ist dieses Verfahren großtechnisch nicht einsetzbar. Zudem müssen die beschichteten Pigmente nach dem Trocknen kalziniert werden, um Farbeffekte zu erzielen, da erst durch die Entfernung des Wassers aus der Metalloxidschicht eine geeignete Schichtstruktur entsteht. Wegen des niedrigen Schmelzpunkts von Aluminium ist das Kalzinieren jedoch bei beschichteten Aluminiumpigmenten nur sehr schlecht möglich.

US-4,978,394 beschreibt die Herstellung Titandioxidbeschichteter Aluminiumpigmente durch chemische Gasphasenbeschichtung (chemical vapor deposition, CVD), wobei Titantetrachlorid in geringer Konzentration mit Wasserdampf in einem fluidisierten Wirbelbett in Gegenwart heißer Aluminiumpartikel umgesetzt wird. Auch bei diesem Verfahren wirkt sich die aufwendige Technologie nachteilig aus.

In US-4,158,074 wird die Herstellung farbiger Aluminiumpigmente durch Beschichten mit einem Film aus hydratisiertem Aluminiumoxid und hydratisiertem Metalloxid offenbart. Der Film wird durch Behandeln feiner Aluminiumplättchen in alkalischer Lösung eines Eisen-, Nickel-, Kobalt-, Zink- oder Kupfersalzes bei erhöhter Temperatur bei pH 8 bis 12 erzeugt, d.h. duch eine elektrochemische Reaktion der Metallsalze. So lassen sich goldfarbige Pigmente, durch Zusatz von Chelatbildnern auch schwarzbraune und grauweiße Pigmente herstellen.

In US-5,261,955 wird ein Sol-Gel-Prozess zur Herstellung farbiger Metallpigmente beschrieben, wobei die Metallplättchen in einem Sol eines anorganischen Salzes dispergiert werden, z.B. einem wässerigen alkalischen Zirkoniumoxid-Sol, die mit dem Sol beschichteten Plättchen nach Filtration in einer Lösung einer anorganischen Verbindung, z. B. Kobaltnitrat, in einem organischen Lösungsmittel dispergiert werden, und durch Erhitzen eine Sol-Gel-Schicht auf den Plättchen gebildet wird. Durch die Vielzahl von einzelnen Schritten ist auch bei diesem Verfahren ein hoher apparativer Aufwand erforderlich.

JP-A-61-130375 offenbart ein goldfarbenes Aluminiumpigment, hergestellt durch Behandeln von Aluminiumpulver mit Dichromat, Natriumfluorid und oberflächenaktiven Mitteln in saurer Lösung, Trocknen und anschließendes Behandeln mit einem Fettsäurederivat. Andere Farbtöne als Gold lassen sich mit dieser Methode nicht erzielen.

DE-OS 41 40 295 beschreibt Pigmente aus plättchenförmigen Trägermaterialien, bevorzugt Glimmer, die von einer anorganischen Matrix überzogen sind, in der Metalloxide und/oder Farbstoffpartikel im Submikrometerbereich enthalten sind. Die Beschichtung der Substrate erfolgt aus sauren wässerigen Suspensionen durch Hydrolyse von Metallsalzen, bevorzugt Titantetrachlorid, in Gegenwart von Metalloxid- und/oder Farbstoffpartikeln. Die Einfärbung von Aluminiumplättchen nach diesem Verfahren ist jedoch nicht möglich, weil sich die Aluminiumpartikel unter diesen Bedingungen rasch zersetzen.

US-3,067,052 offenbart farbige Aluminiumpigmente, die durch Oxidation von Aluminiumpulver mit $KMnO_4$-Lösung gegebenenfalls unter Zusatz eines Reduktionsmittels hergestellt werden. Der Farbton dieser Pigmente ist golden, gegebenenfalls je nach verwendetem Reduktionsmittel auch mit grünlichem oder rötlichem Ton.

Aus DE 25 57 796 ist ein farbiges Aluminiumpigment bekannt, das mit einer Ruß als Farbpigment enthaltenen Metalloxidschicht beschichtet ist. Der Farbpigmentanteil beträgt maximal 10 Gew.-%. Laut dieser Schrift wirken sich größere Mengen an Farbpigment nachteilig auf den Glanz und die Farbe aus.

DE 36 17 430 offenbart beschichtete farbige Pigmente, wobei das Basispigment aus Glimmer besteht. Farbpigmenthaltige Beschichtungen sind in diesem Dokument nicht genannt. Die Farbeffekte der beschriebenen Pigment werden durch Interferenz bewirkt.

DE 42 23 383 beschreibt metallsulfid-beschichtete Substrate, wobei die Metall-Sulfidschicht keine Farbpigmente enthält.

Aus DE 42 23 384 sind metalloxid-beschichtete Alumuiniumsubstrate ohne Farbpigmentanteil bekannt. Für die Erzielung guter Glanzeffekte ist diese Druckschrift zufolge ein möglichst niedriger Gehalt an Kohlenstoff, d.h. an Schmierstoffen und organischen Verunreinigungen entscheidend. Die Substrate müssen daher durch Erhitzen in einer sauerstoffhaltigen Atmosphäre vorbehandelt werden.

JP-1-110 568 (Patent Abstracts of Japan, Sektion C Vol. 13 (1989) Nr. 331 (C-622) offenbart mit dünnen Oxidschichten beschichtete Aluminium-Substrate ohne Farbpigmentanteil, deren Farbeffekte durch Interferenz bewirkt werden.

Die US 3,026,220 beschreibt Pigmente, bei denen das färbende Material in der Überzugsschicht eingeschlossen vorliegt, die hauptsächlich aus Oxid besteht und auf der durch die Peroxidaktivierung entstandenden hydratisierten Alumuminiumoxidschicht angeordnet ist. Diese Pigmente weisen daher einen Aufbau in der Abfolge 1.) Aluminium 2.) nicht eingefärbte Oxid- bzw. Hydroxidschicht und 3.) gefärbte Oxidschicht auf.

Aus der EP 0 571 836 sind Glanzpigmente bekannt, d.h. Pigmente, deren optische Wirkung sich auf die gerichtete Reflexion an den metallischen Grenzflächen zurückführen läßt, bei denen die farbgebende Schicht als diskrete Schicht aus Kohlenstoff, Metall und/oder Metalloxid über der oder zwischen den Metalloxidschicht(en) vorliegt, z.B. als Eisen- oder Chromatschicht. Eine homogene Verteilung der Farbpigmente ist in diesem Dokument nicht offenbart.

Mit den Verfahren zur Herstellung farbiger Pigmente auf Glimmer-Basis lassen sich aus den genannten Gründen keine Aluminiumpigmente herstellen, an denen jedoch wegen der höheren Deckkraft und des Metallglanzes ein großes Interesse besteht. Die bekannten Verfahren zur Einfärbung von Aluminiumpigmenten liefern jedoch nur wenige Farbtöne, überwiegend im Goldbereich, und sind größtenteils apparativ sehr aufwendig. Es bestand daher nach wie vor ein Bedarf an in verschiedenen Farbtönen gefärbten Aluminiumpigmenten und an einem apparativ einfachen Verfahren zur Herstellung dieser farbigen Aluminiumpigmente.

Die Aufgabe der Erfindung besteht darin, solche Pigmente und ein Verfahren zu ihrer Herstellung zur Verfügung zu stellen.

Diese Aufgabe wird gelöst, indem Aluminiumpigmente zur Verfügung gestellt werden, die aus plättchenförmigen Aluminiumsubstraten bestehen, die mit einer farbpigmenthaltigen Metalloxidschicht beschichtet sind. Die erfindungsgemäßen Pigmente werden aus plättchenförmigen Aluminiumsubstraten hergestellt, indem die Metalloxidschicht nicht durch Fällung der Metallsalze aus wässeriger Lösung, sondern durch kontrollierte Hydrolyse von Metallsäureestern in Gegenwart von Farbpigmenten in einem organischen Lösungsmittel mittels eines in einem Schritt durchgeführten Sol-Gel-Prozesses durchgeführt wird. Die erfindungsgemäßen Aluminiumpigmente zeigen verschiedenste Farbtöne, so z.B. Blau, Rot, Violett und Gold, und metallischen Glanz.

Im erfindungsgemäßen Pigment gemäß Anspruch 1 beträgt die Menge an Farbpigment 15 bis 40 Gew.-%. Bevorzugt sind mindestens 20 Gew.-% und die Menge an Metalloxid bevorzugt 3 bis 95 Gew.-%, jeweils bezogen auf das Aluminiumsubstrat.

Die bevorzugten Ausführungsformen sind in den Ansprüchen 2 bis 14 definiert, die Verwendung des Pigments nach Anspruch 1 bis 14 ist in Anspruch 15 beansprucht.

Eine Ausführungsform der Erfindung betrifft farbige Aluminiumpigmente, die dadurch erhältlich sind, daß man

a) eine oder mehrere Farbpigmenttypen auf übliche Art und Weise anreibt,

b) die Anreibung mit Aluminiumpigmenten und einem oder mehreren organischen Lösungsmitteln mischt,

c) einen oder mehrere Metallsäureester zugibt,

d) das 1- bis 5-fache der zur vollständigen Hydrolyse der Metallsäureester stöchiometrisch notwendigen Menge Wasser zugibt,

e) nach Reaktionsende die flüchtigen Bestandteile auf übliche Art aus der Mischung entfernt und

f) die erhaltenen Pigmente trocknet.

In einer bevorzugten Ausführungsform werden die Aluminiumpigmente vor der Durchführung von Schritt b) keiner Entfettungsbehandlung unterzogen.

In einer weiteren bevorzugten Ausführungsform wird ein Teil des Metallsäureesters bereits in Schritt a) während des Verreibens zu dem/den Farbpigmenten gegeben, und entsprechend in Schritt c) die Menge an Metallsäureester verringert.

In einer weiteren Ausführungsform wird in Schritt a) ein übliches Additiv zugesetzt, welches die Pigmentdispergierung verbessert.

Die Erfindung betrifft neben den Pigmenten selbst auch ein Verfahren zu ihrer Herstellung, umfassend die oben erwähnten Schritte a) bis f).

In einer weiteren Ausführungsform des erfindungsgemäßen Verfahrens wird die in Schritt d) erhaltene Reaktionsmischung auf eine Temperatur zwischen 40 °C und dem Siedepunkt des in Schritt b) zugesetzten Lösungsmittels erwärmt und/oder ein basischer Katalysator zugesetzt.

Das Trocknen in Schritt f) erfolgt bevorzugt bei weniger als 200 °C, noch bevorzugter bei weniger als 150 °C, und am günstigsten bei weniger als 100 °C im Vakuum, z.B. bei 90 °C in einem Vakuum-Trockenschrank.

Als Farbpigmente können übliche organische und anorganische Farbpigmente eingesetzt werden. Bevorzugt werden solche Farbpigmente verwendet, die eine hohe Transparenz und Farbechtheit aufweisen. Der Fachmann kann sich an der Literatur (z.B. G. Buxbaum, Industrial Inorganic Pigments, VCH-Verlag, Weinheim, 1993 und W. Herbst, K. Hunger, Industrielle Organische Pigmente, VCH-Verlag, Weinheim, 1987) und an den Herstellerempfehlungen orientieren. Bevorzugte Farbpigmente sind z.B. C.I. Pigment Blue 15:3, C.I. Pigment Red 179, C.I. Pigment Red 101 und C.I. Pigment Red 202. Es kann eine einzelne Pigmenttype oder ein Gemisch mehrerer Pigmenttypen verwendet werden, je nach gewünschtem Farbton des erfindungsgemäßen Aluminiumpigments.

Das Anreiben der Farbpigmente erfolgt in üblicher Art und Weise, z.B. in einer Perlmühle oder in einer Mahlkörpermühle, z.B. mit Zirkonoxidkugeln. Die Anreibung kann in einem Teil des Metallsäureesters oder in einem üblichen Lösungsmittel, z.B. Testbenzin, erfolgen. Auch gleichzeitige Verwendung von Testbenzin oder anderem Lösungsmittel und Metallsäureester beim Anreiben ist möglich. Es ist auch möglich, ein Additiv zur Verbesserung der Pigmentdispergierbarkeit mit zuzugeben, wie z.B. Antiterra U 80, Fa. Byk-Chemie.

Geeignete Aluminiumpigmente in Stufe c) sind alle üblichen für dekorative Beschichtungen einsetzbaren Aluminiumpigmente. Bevorzugt werden runde Aluminiumplättchen verwendet (sog. Silberdollars). Besonders bevorzugt ist die Stapa Metallux 2000-er Typenreihe (Fa. Eckart). Diese Silberdollars ermöglichen wegen ihres geringen Streuanteils besonders brillante Einfärbungen.

Als Lösungsmittel in Stufe b) kommen organische Lösungsmittel in Frage, bevorzugt wassermischbare Lösungsmittel. Besonders bevorzugt sind Alkohole wie Methanol, Ethanol, n-Propanol, i-Propanol, n-Butanol, i-Butanol und t-Butanol, am bevorzugtesten i-Propanol.

Es ist auch möglich, je nach Bedarf nach Stufe d) des Verfahrens vor Reaktionsende erneut eine zusätzliche Menge des in Stufe b) eingesetzten Lösungsmittels zuzugeben. Die Gesamtmenge des zugesetzten Lösungsmittels beträgt bevorzugt 150 bis 300 ml bezogen auf 100 g Aluminiumsubstrat.

Geeignete Metallsäureester werden aus der Alkyl- und Aryl-Alkoholate, Carboxylate, mit Carboxylresten oder Alkylresten oder Arylresten substituierte Alkylalkoholate oder Carboxylate von Titan, Zirkonium, Silicium, Aluminium und Bor umfassenden Gruppe ausgewählt. Besonders bevorzugt sind Alkyl- und Arylalkoholate, insbesondere $C_1$-$C_6$-Alkylalkoholate wie Methanolate, Ethanolate, n-Propanolate, i-Propanolate, n-Butanolate, i-Butanolate und t-Butanolate der genannten Metalle. Diese Verbindungen weisen die allgemeine Formel $M(OR)_y$ auf, wobei M Titan, Zirkonium, Vanadium, Silicium, Aluminium oder Bor ist, R eine $C_1$-$C_6$-Alkyl, Phenyl-, Xylyl, Tolyl- oder Kresylgruppe bedeutet und y 3 oder 4 ist. Dieser Verbindungstyp kann auch als Ester der Metallsäuren, z.B. ortho-Kieselsäure, Borsäure, Aluminiumhydroxid, Titansäure oder Zirkonsäure aufgefaßt werden. Bevorzugt werden Aluminiumtriisopropylat (Triisopropylaluminat), Titantetraisopropylat (Tetraisopropyltitanat), polymeres n-Butyltitanat, Titantetraisobutylat (Tetraisobutyltitanat), Zirkoniumtetraisopropylat (Tetraisopropylzirkonat), o-Kieselsäuretetraethylester (Tetraethylorthosilikat) und Triethylborat (Borsäuretriethylester) verwendet. Auch gemischte Alkoholate sind möglich, d.h. nicht alle Reste OR sind gleich. Darüberhinaus können bevorzugt Acetylacetonate, Acetoacetylacetonate, gegebenenfalls auch durch Alkyl- oder Alkenylreste substituiert, oder Acetoacetate der genannten Metalle eingesetzt werden. Auch gemischte Alkoholate/ Acetylacetonate, Alkoholate/Acetoacetylacetonate oder Alkoholate/Acetoacetate, d.h. solche Metallsäureester, die sowohl Alkoholatreste als auch Acetylacetonatreste, Acetoacetylacetonate oder Acetoacetatreste enthalten, sind geeignet. Bevorzugte Beispiele dieses Metallsäureestertyps sind Zirkonium-, Aluminium- oder Titanacetylacetonat (Zr $(acac)_4$, Ti$(acac)_4$ oder Al$(acac)_3$) und Diisobutyloleylacetoacetylaluminat oder Diisopropyloleylacetoacetylacetonat. Darüber hinaus können auch Mischungen von Metallsäureestern verschiedener Metalle verwendet werden, die auch teilweise kondensiert sein können, beispielsweise Dynasil[R] (Fa. Hüls), ein gemischter Al-Si-Metallsäureester.

Es werden bezogen auf 100 g Aluminiumsubstrat mit einer Oberfläche von ca. 4 $m^2$/g (BET) 15 bis 40 g Farbpigment, bevorzugt 20 bis 40 g, und 0,1 bis 0,8 Mol Metallsäureester, bevorzugt 0,5 Mol, eingesetzt. Bei Verwendung von mehr als 0,8 Mol Metallsäureester erhält man ein farbiges Aluminiumpigment mit schlechtem Glanz und die Pigmente tendieren zu unerwünschter Agglomeration. Bei Verwendung von weniger als 0,1 Mol Metallsäureester reicht die Stabilität des beschichteten Pigments gegen Wasser und Säuren nicht aus und es läßt sich keine ausreichende Haftung der Farbpigmente auf den Aluminiumpigmenten erzielen. Verwendet man weniger Farbpigment als 15 g, so erzielt man keinen ausreichenden Farbeffekt, bei Verwendung von mehr als 40 g Farbpigment wird der Metallglanz der Aluminiumpigmente zu stark überdeckt.

Entscheidend für das erfindungsgemäße Verfahren ist die in Schritt d) eingesetzte Wassermenge, die das 1- bis 5-fache der zur vollständigen Hydrolyse des Metallsäureesters notwendigen Menge beträgt. Ein größerer Wasserüberschuß kann zu Vergrauung der Aluminiumpigmente führen und ist daher zu vermeiden. Bezogen auf 1 Mol eines vierwertigen Metallsäureesters, z.B. eines ortho-Silikats, Titanats oder Zirkonats, werden daher 4 bis 20 Mol Wasser eingesetzt, bevorzugt 4,5 bis 10 Mol. Bezogen auf 1 Mol eines dreiwertigen Metallsäureesters, z.B. eines Borats oder Aluminats, werden entsprechend 3 bis 15 Mol Wasser verwendet, bevorzugt 3,1 bis 8 Mol. Bevorzugt wird vollentsalztes Wasser eingesetzt.

Die Reaktion wird bei einer Temperatur von Raumtemperatur bis zum Siedepunkt des/der Lösungsmittel durchgeführt. Die Temperatur richtet sich nach der Reaktivität der Metallsäureesters und wird nach Bedarf eingestellt.

Insbesondere bei Verwendung von Kieselsäureestern ist die Zugabe basischer Katalysatoren bevorzugt, die bei Schritt d) zugegeben werden können. Es können übliche basische Katalysatoren verwendet werden. Geeignete Basen sind z.B. Amine wie Triethylamin, Ethylendiamin oder Tributylamin oder substituierte Amine wie Dimethylethanolamin oder Methoxypropylamin. Darüber hinaus ist es auch möglich, autokatalytisch wirkende Aminosilane zuzusetzen, z. B. 3-Aminopropyl-trimethoxysilan, N-Aminoethyl-3-aminopropyltrimethoxysilan oder 3,4,5-Dihydroimidazol-1-yl-propyltriethoxysilan. Gegebenenfalls kann nach einiger Zeit nochmals eine weitere Basenzugabe erfolgen.

Nach Beendigung der Reaktion, d.h. in der Regel nach 0,5 bis 10 Stunden, werden die flüchtigen Bestandteile auf übliche Art aus dem Reaktionsgemisch entfernt, z.B. durch Destillation oder durch Abpressen. Anschließend werden die Pigmente getrocknet, z.B. in einem Vakuum-Trockenschrank.

Die farbigen Aluminiumpigmente können wie übliche Aluminiumpigmente weiterverarbeitet werden. Beispielsweise ist es für viele Anwendungsbereiche vorteilhaft, durch Zugabe von Lösungsmitteln wie Kohlenwasserstoffen, Ethylacetat, Methoxypropanol oder Butylglykol nichtstaubende Pasten herzustellen.

Beim erfindungsgemäßen Herstellungsverfahren für farbige Aluminiumpigmente wird der Metallsäureester hydrolysiert und anschließend kondensiert, wobei in einem Sol-Gel-Prozeß letztendlich Metalloxide gebildet werden, die sich auf den Aluminiumplättchen in Form einer dichten, geschlossenen Beschichtung abscheiden und dabei gleichzeitig die Farbpigmente auf der Metalloberfläche fixieren. Die Hydrolyse/Kondensation verläuft auf sehr komplexe Art und Weise, für Alkoholat-Metallsäureester läßt sich der Beginn der Reaktionskette durch folgende Gleichungen darstellen:

$$(1) \qquad M(OR)_y + H_2O \rightarrow (RO)_{y-1}\text{-M-OH} + ROH$$

$$(2) \qquad 2\,(RO)_{y-1}\text{-M-OH} \rightarrow (RO)_{y-1}\text{-M-O-M-}(OR)_{y-1}$$

Eine Funktion der Metalloxidschicht liegt darin, als "Klebstoff" für einen haftfesten Verbund zwischen Metallpigment und Farbpigment zu sorgen. Eine weitere Funktion der Metalloxidschicht besteht darin, das Aluminiumpigment vor Feuchtigkeit und Chemikalien zu schützen. Die erfindungsgemäßen farbigen Aluminiumpigmente sind daher z.B. sowohl in konventionellen lösemittelhaltigen Lacken als auch in Wasserlacken einsetzbar.

Die erfindungsgemäßen neuen, farbigen Aluminiumpigmente weisen eine besonders große Brillianz und ein breites Spektrum an möglichen Farbnuancen auf. Die Pigmente sind sehr stabil. Das Verfahren ist einfach auch großtechnisch durchzuführen und erfordert keine aufwendigen Apparaturen. Ein Kalzinieren der Pigmente ist nicht notwendig, da die erfindungsgemäßen Pigmente bereits nach dem Trocknen eine hervorragende Brillanz, Farbintensität und Stabilität zeigen.

Die erfindungsgemäßen Pigmente können vorteilhaft zur Effektgebung in Lacken, Beschichtungen, Kunststoffen, Druckfarben und kosmetischen Zubereitungen eingesetzt werden.

Im folgenden wird die Erfindung an Hand von Beispielen erläutert.

Beispiel 1

30 g C.I. Pigment Blue 15:3 werden mit 85 g Kieselsäuretetraethylester (Fa. Wacker) mit Hilfe von 300 g Zirkonoxidkugeln (0,7 mm Durchmesser) in einer Mahlkörpermühle (Red Devil, Fa. Union N.J. USA) dispergiert und gemahlen. Zu dieser Dispersion gibt man bei Raumtemperatur 100 g Aluminiumpigment (Stapa Metallux 2154, BET-Oberfläche 3,8 m$^2$/g, Fa. Eckart) und 208 ml Isopropanol und rührt das Gemisch eine Stunde lang. Danach gibt man eine Lösung aus 0,78 g Ethylendiamin und 51,4 g vollentsalztem Wasser zu und erhitzt die Suspension unter Rühren auf 80 °C. Im Abstand von je einer Stunde werden noch zweimal je 0,78 g Ethylendiamin und 51,4 g Isopropanol zugegeben. Das Gemisch wird insgesamt während 6 Stunden erhitzt. Anschließend wird das Produkt abfiltriert und bei 90 °C im Vakuumschrank getrocknet. Man erhält ein metallisch glänzendes, intensiv blaues Aluminiumpigment.
Farbpigment: ca. 19 %, $S_iO_2$: ca. 15 %;
Aluminiumgehalt: ca. 65 %

Beispiel 2

20 g C.I. Pigment Red 179 werden mit 56,7 g Kieselsäuretetraethylester, 23,3 g Testbenzin und 11,4 g eines Netz- und Dispergieradditivs (Antiterra U 80, Byk-Chemie) nach dem Verfahren aus Beispiel 1 gemahlen. Zu dem Gemisch werden 100 g Aluminiumpigment (Stapa Metallux 2154) und 208 ml Isopropanol gegeben. Nach 0,5 Stunden Rühren bei Raumtemperatur werden 28,3 g Kieselsäuretetraethylester zugegeben. Nach weiteren 0,5 Stunden wird eine Lösung aus 0,78 g Ethylendiamin und 51,4 g vollentsalztem Wasser zugegegeben und auf 80 °C erwärmt. Im Abstand von 1 Stunde werden noch zweimal je 0,78 g Ethylendiamin und 51,4 g Isopropanol zugegeben. Nach 6-stündigem Erwärmen wird das Gemisch abfiltriert und bei 90 °C im Vakuumschrank getrocknet. Man erhält ein metallisch glänzendes dunkelrotes Aluminiumpigment.
Farbpigment: ca. 15 %; $S_iO_2$: ca. 17 %;
Aluminiumgehalt: ca. 69 %

Beispiel 3

30 g C.I. Pigment Blue 15:3 werden in 85 g Testbenzin entsprechend Beispiel 1 gemahlen. 100 g Aluminiumpigment (Stapa Metallux 2154, Fa. Eckart) und 208 ml Isopropanol werden dazugemischt. Nach 0,5 Stunden werden 85 g Kieselsäuretetraethylester zugegeben. Nach weiteren 0,5 Stunden wird nach Zugabe einer Lösung aus 0,78 g Ethylendiamin in 51,4 g vollentsalztem Wasser auf 80 °C aufgeheizt. Nach zwei Stunden werden 0,78 g Ethylendiamin und 51,4 g Isopropanol zugegeben. Nach 6-stündigem Erhitzen wird das Gemisch abfiltriert und bei 90 °C im Vakuumschrank getrocknet. Man erhält ein blaues, metallisch glänzendes Aluminiumpigment.
Farbpigment: ca. 19 %; $S_iO_2$: ca. 15 %;
Aluminiumgehalt: ca. 65 %

Beispiel 4

40 g C.I. Pigment Red 101 werden in 90 g Isopropanol wie in Beispiel 1 gemahlen. Das weitere Vorgehen entspricht dem von Beispiel 3. Als Aluminiumpigment wird STAPA Metallux 8154 der Anmelderin verwendet. Man erhält ein metallisch glänzendes, goldenes Aluminiumpigment.
Farbpigment: ca. 24 %; $S_iO_2$: ca. 14 %
Aluminiumgehalt: ca. 61 %

Beispiele 5 bis 8

In den Beispielen 5 bis 8 erfolgt die Dispergierung des jeweiligen Farbpigments entsprechend Beispiel 3. Die Einfärbung erfolgt ebenfalls wie in Beispiel 3 mit den in Tabelle 1 angegebenen Metallsäureestern und den in der Tabelle 1 aufgeführten Wassermengen.

**TABELLE 1**

| | Beispiel 5 | Beispiel 6 | Beispiel 7 | Beispiel 8 |
|---|---|---|---|---|
| Farbpigment C.I. | Pigment Red 202 | Pigment Red 179 | Pigment Red 202 | Pigment Red 202 |
| Metallsäureester (jewells 85 g) | Zirkon-acetyl-acetonat | Borsäuretri-ethylester | Diisobutyloleyl-acetoacetylaluminat | Titan(IV)-iso-propylat |
| Lieferant | ALDRICH | ALDRICH | KENRICH | KENRICH |
| Wassermenge in g | 48 | 32,4 | 34,3 | 26,7 |
| eingefärbtes Aluminiumpigment | metallisch glänzend rot-violett | metallisch glänzend rot | metallisch glänzend rot-violett | metallisch glänzend rot-violett |
| Zusammensetzung: Farbpigment | 19,60% | 16,20% | 18,50% | 18,10% |
| Metalloxid | 11,4% $ZrO_2$ | 21,2% $B_2O_3$ | 9,3% $Al_2O_3$ | 13,5% $TiO_2$ |
| Aluminium | 69% | 62,30% | 71,20% | 67,40% |

**Patentansprüche**

1. Farbige Aluminiumpigmente, umfassend plättchenförmige, mit einer einzigen Metalloxidschicht beschichtete Alwminiumsubstrate,
**dadurch gekennzeichnet,**
daß die Metalloxidschicht Farbpigmente in einer Menge von 15 bis 40 Gew.% bezogen auf das Aluminumsubstrat

7

umfaßt, und die Farbpigmente nicht in Form einer Schicht aus Kohlenstoff, Metall und/oder Metalloxid vorliegen.

2. Farbige Aluminiumpigmente nach Anspruch 1,
   **dadurch gekennzeichnet,**
   daß die Menge an Metalloxid 3 bis 95 Gew.% beträgt, bezogen auf das Aluminiumsubstrat.

3. Farbige Aluminiumpigmente nach Anspruch 1,
   **dadurch gekennzeichnet,**
   daß der Farbpigmentanteil in der Metalloxidschicht mindestens 20 bis höchstens 40 Gew.% bezogen auf das Aluminiumsubstrat beträgt.

4. Farbige Aluminiumpigmente nach Anspruch 2 oder 3,
   **dadurch gekennzeichnet,**
   daß es sich bei den Farbpigmenten um organische Farbpigmente handelt.

5. Farbige Aluminiumpigmente nach einem der Ansprüche 1 bis 4,
   **dadurch erhältlich,**
   daß man

   a) eine oder mehrere Farbpigmenttypen auf übliche Art und Weise anreibt,
   b) die Anreibung mit Aluminiumgigmenten und einem oder mehreren organischen Lösungsmitteln mischt,
   c) einen oder mehrere Metallsäureester zugibt,
   d) das 1- bis 5-fache der zur vollständigen Hydrolyse der Metallsäureester stöchiometrisch notwendigen Menge Wasser zugibt,
   e) nach Reaktionsende die flüchtigen Bestandteile auf übliche Art aus der Mischung entfernt und
   f) die erhaltenen Pigmente trocknet.

6. Farbige Aluminiumpigmente nach Anspruch 5,
   **dadurch erhältlich,**
   daß die Aliminiumpigmente vor der Durchführung von Schritt b) keiner Entfettungsbehandlung unterzogen - wurden.

7. Aluminiumpigmente nach Anspruch 5 oder 6,
   **dadurch erhältlich,**
   daß man beim Anreiben der Farbpigmente in Schritt a) einen Teil der Gesamtmenge an Metallsäureester zugibt und dann die Schritte b) bis f) durchführt, wobei in Schritt c) entsprechend weniger Metallsäureester zugegeben wird.

8. Aluminiumpigmente nach einem der Ansprüche 5 bis 7,
   **dadurch erhältlich,**
   daß in Schritt a) ein Additiv zur Verbesserung der Pigmentdispergierung zugesetzt wird.

9. Aluminiumpigmente nach einem oder mehreren der Ansprüche 5 bis 8,
   **dadurch erhältlich,**
   daß bezogen auf 100 g Aluminiumsubstrat des Schritts b)
   15 bis 40 g Farbpigment und insgesamt 0,1 bis 0,8 Mol Metallsäureester eingesetzt werden.

10. Aluminiumpigmente nach einem oder mehreren der Ansprüche 5 bis 9,
    **dadurch erhältlich,**
    daß der/die Metallsäureester aus der Alkylalkoholate, Arylalkoholate, Carboxylate, mit Carboxylresten oder Alkylresten oder Arylresten substituierte Alkoholate oder Carboxylate von Titan, Zirkonium, Vanadium, Silicium, Aluminium und Bor oder gemischte kondensierte Metallsäureester dieser Metalle umfassenden Gruppe ausgewählt wird/werden.

11. Aluminiumpigmente nach einem oder mehreren der Ansprüche 5 bis 10,
    **dadurch erhältlich,**
    daß der/die Metallsäureester aus der Triisopropylaluminat, Tetraisopropyltitanat, Tetraisobutyltitanat, polymeres n-Butyltitanat, Tetraisopropylzirkonat, Tetraethylorthosilikat, Triethylborat, Aluminiumacetylacetonat, Titanacetylacetonat, Zirkoniumacetylacetonat, Diisobutyloleylacetoacetylaluminat, Diisoprogyloleylacetoacetylacetonat oder gemischte Si-Al-Metallsäureester umfassenden Gruppe ausgewählt wird/werden.

12. Aluminiumpigmente nach einem oder mehreren der Ansprüche 5 bis 11,
    **dadurch erhältlich,**
    daß man die in Schritt d) erhaltene Reaktionsmischung auf eine Temperatur von 40 °C bis zum Siedepunkt des Lösungsmittels erwärmt und/oder einen basischen Katalysator zugibt und anschließend die Schritte e) und f) durchführt.

13. Aluminiumpigmente nach einem oder mehreren der Ansprüche 5 bis 12,
    **dadurch erhältlich,**
    daß man in Schritt f) bei weniger als 200 °C trocknet.

14. Aluminiumpigmente nach einem oder mehreren der Ansprüche 5 bis 13,
    **dadurch erhältlich,**
    daß man in Schritt f) bei weniger als 100 °C im Vakuum trocknet.

15. Verwendung der Aluminiumpigmente nach einem oder mehreren der Ansprüche 1 bis 14 als Effektpigmente in - Lacken, Beschichtungen, Druckfarben, Kunststoffen und kosmetischen Zubereitungen.

## Claims

1. Coloured aluminium pigments comprising platelet-shaped aluminium substrates coated with a single metal-oxide layer, characterized in that the metal-oxide layer comprises colour pigments in an amount of from 15 to 40% by weight, based on the aluminium substrate, and the colour pigments are not in the form of a layer of carbon, metal and/or metal oxide.

2. Coloured aluminium pigments according to Claim 1, characterized in that the amount of metal oxide is from 3 to 95% by weight, based on the aluminium substrate.

3. Coloured aluminium pigments according to Claim 1, characterized in that the proportion of colour pigment in the metal-oxide layer is from at least 20 to not more than 40% by weight, based on the aluminium substrate.

4. Coloured aluminium pigments according to Claim 2 or 3, characterized in that the colour pigments are organic colour pigments.

5. Coloured aluminium pigments according to one of Claims 1 to 4, obtainable by

    a) subjecting one or more types of colour pigments to conventional dispersion,
    b) mixing the dispersion with aluminium pigments and with one or more organic solvents,
    c) adding one or more metal acid esters,
    d) adding from one to five times the amount of water required by stoichiometry for complete hydrolysis of the metal acid esters,
    e) after the end of reaction, removing the volatile constituents from the mixture by conventional means, and
    f) drying the resulting pigments.

6. Coloured aluminium pigments according to Claim 5, obtainable by not subjecting the aluminium pigments to any degreasing treatment prior to the performance of step b).

7. Aluminium pigments according to Claim 5 or 6, obtainable by adding, in the course of dispersing the colour pigments in step a), a portion of the overall amount of metal acid esters and then performing steps b) to f), with the addition in step c) of correspondingly less metal acid ester.

8. Aluminium pigments according to one of Claims 5 to 7, obtainable by adding, in step a), an additive for improving pigment dispersion.

9. Aluminium pigments according to one or more of Claims 5 to 8, obtainable by employing from 15 to 40 g of colour pigment and a total of from 0.1 to 0.8 mol of metal acid ester per 100 g of the aluminium substrate of step b).

10. Aluminium pigments according to one or more of Claims 5 to 9, obtainable by selecting the metal acid ester(s) from the group consisting of alkyl alcoholates, aryl alcoholates, carboxylates, carboxyl-, alkyl- or aryl-substituted alcoholates or carboxylates of titanium, zirconium, vanadium, silicon, aluminium and boron, or mixed condensed metal acid esters of these metals.

11. Aluminium pigments according to one or more of Claims 5 to 10, obtainable by selecting the metal acid ester(s) from the group consisting of triisopropyl aluminate, tetraisopropyl titanate, tetraisobutyl titanate, polymeric n-butyl titanate, tetraisopropyl zirconate, tetraethyl orthosilicate, triethyl borate, aluminium acetylacetonate, titanium acetylacetonate, zirconium acetylacetonate, diisobutyl oleyl acetoacetyl aluminate, diisopropyl oleyl acetoacetyl acetoneate, or mixed Si-Al metal acid esters.

12. Aluminium pigments according to one or more of claims 5 to 11, obtainable by heating the reaction mixture obtained in step d) to a temperature from 40°C to the boiling point of the solvent and/or adding a basic catalyst and then performing steps e) and f).

13. Aluminium pigments according to one or more of Claims 5 to 12, obtainable by drying at less than 200°C in step f).

14. Aluminium pigments according to one or more of claims 5 to 13, obtainable by drying at less than 100°C under vacuum in step f).

15. Use of the aluminium pigments according to one or more of Claims 1 to 14 as special-effect pigments in lacquers, coatings, printing inks, plastics and cosmetic preparations.

## Revendications

1. Pigments d'aluminium colorés comprenant un substrat d'aluminium en forme de plaquettes, recouvert d'une couche unique d'oxyde,
   **caractérisés en ce que**
   les pigments, colorés formant la couche d'oxyde représentent 15 à 40% par rapport au substrat d'aluminium et en ce que les pigments colorés ne sont pas sous forme d'une couche de carbone, de métal et/ou d'oxyde métallique.

2. Pigments d'aluminium colorés selon la revendication 1,
   **caractérisés en ce que**
   la quantité d'oxyde métallique représente 3 à 95% par rapport au substrat d'aluminium.

3. Pigments d'aluminium colorés selon la revendication 1,
   **caractérisés en ce que**
   la portion de pigments colorés dans la couche d'oxyde métallique représente au moins 20 jusqu'à 40% au plus par rapport au substrat d'aluminium.

4. Pigments d'aluminium colorés selon la revendication 2 ou 3,
   **caractérisés en ce que**
   les pigments colorés sont des pigments colorés organiques.

5. Pigments d'aluminium colorés selon l'une des revendications 1 à 4,
   **caractérisés en ce qu'**
   on l'obtient par le processus suivant :

   a) on broie un ou plusieurs types de pigments de manière usuelle;
   b) on mélange le produit du broyage avec des pigments d'aluminium et un ou plusieurs agents solvants organiques ;
   c) on ajoute un ou plusieurs esters d'acides métalliques ;
   d) on ajoute 1 à 5 fois la quantité stoechiométrique d'eau nécessaire à l'hydrolyse complète de l'ester d'acide métallique ;
   e) à l'issue de la réaction on élimine de façon usuelle les constituants volatils du mélange; et

f) on sèche les pigments obtenus.

6. Pigments d'aluminium colorés selon la revendication 5,
**caractérisés en ce que**
les pigments d'aluminium ne subissent aucun dégraissage avant la réalisation de l'étape b).

7. Pigments d'aluminium colorés selon la revendication 5 ou 6,
**caractérisés en ce que**
lors du broyage des pigments colorés à l'étape a) on ajoute une fraction de la quantité totale de l'ester d'acide métallique, puis on réalise les étapes b) à f), de sorte qu'à l'étape c), on ajoute une quantité réduite de façon correspondante de l'ester d'acide métallique.

8. Pigments d'aluminium colorés selon l'une des revendications 5 à 7,
**caractérisés en ce que**,
à l'étape a), on ajoute un additif pour améliorer la dispersion des pigments.

9. Pigments d'aluminium colorés selon l'une ou plusieurs des revendications 5 à 8,
**caractérisés en ce que**,
rapporté à 100 g de substrat d'aluminium à l'étape b), on ajoute 15 à 40 g de pigment coloré, et un total de 0,1 à 0,8 mol d'ester d'acide métallique.

10. Pigments d'aluminium colorés selon l'une ou plusieurs des revendications 5 à 9,
**caractérisés en ce que**
l' / les ester(s) d'acide(s) métallique(s) est / sont choisi(s) dans le groupe constitué par les alcoolates d'alkyle, les alcoolates d'aryle, les carboxylates, les alcoolates ou carboxylates substitués par des restes carboxyle, alkyle ou aryle, de titane, de zirconium, de ranadium, de silicium, d'aluminium ou de bore, ou des esters condensés mixtes d'acides métalliques de ces métaux.

11. Pigments d'aluminium colorés selon l'une ou plusieurs des revendications 5 à 10,
**caractérisés en ce que**
l' / les ester(s) d'acide(s) métallique(s) est / sont choisi(s) dans le groupe constitué par l'aluminate de tri-isopropyle, le titanate de tétra-isopropyle, le titanate de tétra-isobutyle, les polymères de titanate de n-butyle, de zirconate de tétra-isopropyle, de l'orthosilicate de tétraéthyle, de borate de triéthyle, d'acétylacétonate d'aluminium, l'acétylacétonate de titane, l'acétylacétonate de zirconium, l'acétoacétylaluminate de di-isobutyloléyle, l'acétoacétylacétonate de di-isopropyloéyle ou des esters mixtes d'acides métalliques du Si et du Al.

12. Pigments d'aluminium colorés selon l'une ou plusieurs des revendications 5 à 11,
**caractérisés en ce que**
l'on chauffe le mélange réactionnel obtenu à l'étape d) à une température allant de 40°C jusqu'au point d'ébullition de l'agent solvant, et/ou on ajoute un catalyseur basique, et enfin on conduit les étapes e) et f).

13. Pigments d'aluminium colorés selon l'une ou plusieurs des revendications 5 à 12,
**caractérisés en ce que**
le séchage à l'étape f) est réalisé à une température inférieure à 200°C.

14. Pigments d'aluminium colorés selon l'une ou plusieurs des revendications 5 à 13,
**caractérisés en ce que**
le séchage à l'étape f) est conduit sous vide à une température inférieure à 100°C.

15. Utilisation des pigments d'aluminium selon l'une ou plusieurs des revendications 1 à 14 comme pigments générateurs d'effets dans des laques, des compositions pour revêtements, des colorants pour l'imprimerie, des matières plastiques et des préparations cosmétiques.